# EUROPEAN PATENT SPECIFICATION

(11) **EP 1 648 562 B1**
(45) Date of publication and mention of the grant of the patent: **07.03.2012**
(21) Application number: 04756854.8
(22) Date of filing: 09.07.2004
(51) Int. Cl.: A61N 5/067

(54) **DEVICE FOR DISTAL PROTECTION AND TREATMENT OF BLOOD VESSELS**
VORRICHTUNG ZUM DISTALEN SCHUTZ UND BEHANDLUNG VON BLUTGEFÄSSEN
DISPOSITIF DE PROTECTION ET DE TRAITEMENT À DISTANCE DE VAISSEAUX SANGUINS

(30) Priority: 09.07.2003 US 486178 P
(43) Date of publication of application: 26.04.2006
(73) Proprietor: Light Sciences Oncology, Inc., Bellevue, WA 98006 (US)
(72) Inventor: CHEN, James, Bellevue, WA 98009 (US); SHINE, David, Sammamish, WA 98074 (US); LICHTTENEGGER, Gary, Woodinville, WA 98072 (US); GUO, Zihong, Bellevue, WA 98006 (US); BURWELL, Phillip, Snohomish, WA 98290 (US); DALY, Steven, Sammamish, WA 98075 (US)
(74) Representative: Grünecker, Kinkeldey, Stockmair & Schwanhäusser
(86) International application number: PCT/US2004/022130
(87) International publication number: WO 2005/007216

(56) References cited:
- EP-A- 0 820 786
- WO-A-96/29943
- WO-A-2004/082736
- US-A- 4 445 892
- US-A- 4 470 407
- US-A- 5 178 616
- US-A- 5 445 608
- US-B1- 6 540 767
- US-B2- 6 562 058
- US-B2- 6 585 655

## Description

### Field of the Invention

The present invention generally relates to a apparatus for using light to diagnose and treat tissue, and more specifically, to a apparatus to diagnose or treat tissue accessible via a cavity, duct, vessel or other body lumen, in connection with a distal protection device.

### Background of the Invention

Photodynamic therapy (PDT) is a process whereby light of a specific wavelength or waveband is administered to tissue, to enable diagnosis or treatment of the tissue. The tissue is rendered photosensitive through the administration of a photoreactive or photosensitizing agent having a characteristic light absorption waveband. In PDT, the photoreactive agent is administered to a patient, typically by intravenous injection, oral administration, or by local delivery to the treatment site. Abnormal tissue in the body is known to selectively absorb certain photoreactive agents to a much greater extent than normal tissue. Once the abnormal tissue has absorbed or linked with the photoreactive agent, the abnormal tissue can then be diagnosed or treated by administering light having a wavelength or waveband corresponding to the absorption wavelength or waveband of the photoreactive agent. The PDT can then cause necrosis of the abnormal tissue.

PDT has proven to be very effective in destroying abnormal tissue, such as cancer cells, and has also been proposed for the treatment of vascular diseases, such as atherosclerosis and restenosis due to intimal hyperplasia. In the past, percutaneous transluminal coronary angioplasty (PTCA) has typically been performed to treat atherosclerotic cardiovascular diseases. A more recent treatment based on the use of drug eluting stents has reduced the rate of restenosis in some diseased vessels. As effective as such therapies are, a new form of therapy is needed for treating peripheral arterial disease and more problematic coronary diseases; such as vulnerable plaque, saphenous vein bypass graft disease, and diffuse long lesions.

As noted above, the objective of PDT may be either diagnostic or therapeutic. In diagnostic applications, the wavelength of light is selected to cause the photoreactive agent to fluoresce, yielding information about the tissue without damaging the tissue. In therapeutic applications, the wavelength/waveband of light delivered to the tissue treated with the photoreactive agent causes the photoreactive agent to undergo a photochemical reaction with oxygen in the localized tissue, which is believed to yield free radical species (such as singlet oxygen) that cause localized cell lysis or necrosis. The central strategy to inhibit arterial restenosis using PDT, for example, is to cause a depletion of vascular smooth muscle cells, which are a source of neointima cell proliferation (see, Nagae et al., Lasers in Surgery and Medicine 28:381-388, 2001). One of the advantages of PDT is that it is a targeted technique, in that selective or preferential delivery of the photoreactive agent to specific tissue enables only the selected tissue to be treated. Preferential localization of a photoreactive agent in areas of arterial injury, with little or no photoreactive agent delivered to healthy portions of the arterial wall, can therefore enable highly specific PDT ablation of arterial tissue.

Light delivery systems for PDT are well known in the art. Delivery of light from a light source such as a laser, to the treatment site has typically been accomplished through the use of a single optical fiber delivery system with special light-diffusing tips affixed thereto. Exemplary prior art devices also include single optical fiber cylindrical diffusers, spherical diffusers, micro-lensing systems, an over-the-wire cylindrical diffusing multi-optical fiber catheter, and a light-diffusing optical fiber guidewire. Such prior art PDT illumination systems generally employ remotely disposed high power lasers or solid state laser diode arrays, which are coupled to optical fibers for delivery of light to a treatment site. The disadvantages of using laser light sources include relatively high capital costs, relatively large size, complex operating procedures, and the safety issues that must be addressed when working with high power lasers. Accordingly, there is a substantial need for a light generating system that does not include a laser, and which generates light at the treatment site instead of at a remote point. For vascular applications of PDT, it would be desirable to provide a light-generating apparatus having a minimal cross-section, a high degree of flexibility, and compatibility with a guidewire introduction system, so the light-generating apparatus can readily be delivered to the treatment site through a vascular lumen. Such an apparatus should also deliver light uniformly to the treatment area.

For vascular application of PDT, it would further be desirable to provide a light-generating apparatus that is easily centered within a blood vessel, and which is configured to prevent light absorbent material, such as blood, from being disposed in the light path between the target tissue and the apparatus. Typically, an inflatable balloon catheter that matches the diameter of the blood vessel when the balloon is inflated is employed for centering apparatus within a vessel. Such devices also desirably occlude blood flow, enabling the light path to remain clear of obstructing blood.

Historically, the saphenous vein has been used to bypass stenotic coronary arteries during a PTCA surgical procedure. Increasing experience with postoperative follow-up of patients after saphenous vein bypass grafting has revealed a significant incidence of saphenous vein graft disease. Vein grafts develop endothelial proliferation as soon as they are placed in arterial circulation and after a few years, tend to develop atherosclerosis with thrombus formation. Vein graft atherosclerosis is often diffuse, concentric, and friable, with a poorly developed fibrous cap. Because of this characteristic, percutaneous interventions in saphenous vein grafts are limited by distal embolization, which can be extremely dangerous to a patient. Several types of catheter systems have been designed to capture atherothrombotic debris that embolize distally during vein graft intervention, where the intervention includes balloon dilation and/or stent placement. A distal protection device typically employs one of two approaches - a distal occlusion with a flow-occlusion balloon, followed by aspiration, and a distal occlusive filter. Neither approach is sufficient by itself. Therefore, it would be desirable to provide additional distal protection, to prevent accelerated vein graft disease, and to prevent distal embolization during interventions.

Post-published WO 2004/082736 A relates to techniques for using light to diagnose and treat tissue accessible via a cavity, duct, or vessel of a body. In one example, a light source array including a plurality of light emitting diodes, a focusing lens and a light-diffusing element are included in a distal end of a catheter. A balloon is optionally provided to interrupt blood flow that can block the transmission of light, and to center the apparatus in a blood vessel. Optical fibers optionally direct light from the light source to the diffusing element. The light source array can have a radial or linear configuration and can produce more than one wavelength of light for activating different photoreactive agents. Linear light source elements are particularly useful for treating elongate portions of tissue in a vessel. One example intended for use with a conventional balloon catheter integrates light sources into a guidewire.

### SUMMARY OF THE INVENTION

It is the object of the present invention to improve prior art systems. This object is solved by the subject matter of the independent claim. Preferred embodiments are defined by the dependent claims.

The present invention encompasses light generating devices for illuminating portions of vascular tissue to administer PDT. Each embodiment includes one or more light sources adapted to be positioned inside a body cavity, a vascular system, or other body lumen. While the term "light source array" is frequently employed herein, because particularly preferred embodiments of this invention include multiple light sources arranged in a radial or linear array, it should be understood that a single light source can also be employed within the scope of this invention. Using a plurality of light sources generally enables larger treatment areas to be illuminated. Light emitting diodes (LEDs) are particularly preferred as light sources, although other types of light sources can be employed, as described in detail below. The light source that is used is selected based on the characteristics of a photoreactive agent with which the apparatus is intended to be used, since light of incorrect wavelengths or waveband will not cause the desired reaction by the photoreactive agent. An array of light sources can include light sources that provide more than one wavelength or produce light that covers a waveband. Linear light source arrays are particularly useful to treat elongate portions of tissue within a lumen. Light source arrays used in this invention can also optionally include reflective elements to enhance the transmission of light in a preferred direction. Each embodiment described herein can beneficially include expandable members to occlude blood flow and to enable the apparatus to be centered in a blood vessel.

A key aspect of the light generating device of the present invention is that each embodiment is either adapted to be used with, or includes, a distal protection device. Interventions on vessels often results in distal embolization of atherosclerotic debris downstream, which can result in clinically significant events, including myocardial infarction, stroke, and renal failure. Distal protection devices trap blood and suspended debris, enabling removal of such debris before unobstructed flow is restored. Studies relating to the use of distal protection devices indicate such devices reduce the incidence of major adverse cardiac events by as much as 50 percent.

The present invention uses at least one of an integrated light source element disposed on a distal end of an intra lumen device, and a substantially transparent hollow shaft disposed on a distal end of an intra lumen device, the hollow shaft being configured to accommodate a separate light source element. When a separate light source element is employed, the separate light source element is advanced through a working lumen in the intra lumen device and into the hollow shaft, after the intra lumen device is properly positioned in a body lumen. Preferably, the present invention also includes a hollow tip disposed distally of the light source element (or of the hollow shaft that is adapted to receive a separate light source element). The hollow tip includes an orifice at its distal end and an orifice on a side surface of the hollow tip, which enable the intra lumen device to be advanced over a guidewire, without the need to extend a guidewire lumen in the light source element (or in the hollow shaft into which a separate light source element will be introduced). A guidewire lumen is preferably included to enable the intra lumen device to be advanced over a guidewire; also preferably included is a flushing and aspiration lumen. The flushing and aspiration lumen enables a flushing fluid to be introduced into an isolated portion of a body lumen and enables the flushing fluid and any debris to be subsequently evacuated (i.e., aspirated) from the isolated portion of the body lumen.

In one embodiment of the present invention, a first intra lumen device does not include a distal protection device, but instead, is adapted to be used with existing distal protection devices. The first intra lumen device includes the light source element (or the hollow shaft adapted to accommodate a separate light source element), the hollow tip, the guidewire lumen, and the flushing lumen, all of which were discussed above. The first intra lumen device is adapted to be used with a guide catheter having an occlusion balloon at its distal end, and a distal protection device. A guidewire, distal protection device, and guide catheter are introduced into a body lumen, so that a distal end of the guidewire is disposed beyond the treatment area, the distal protection device is disposed distal of the treatment area, and a distal end of the guide catheter is disposed proximal of the treatment site. The first intra lumen device is advanced into the body lumen until the distal end of the first intra lumen device (including the light source element or the hollow shaft adapted to accommodate a light source element) is disposed adjacent to the treatment area, and between the distal end of the guide catheter and the distal protection device. If a separate light source element is used, the separate light source element is advanced into the hollow shaft adapted to accommodate the separate light source element. The distal protection device and the guide catheter balloon are activated, isolating the treatment area. Flushing fluid is introduced into the isolated area to displace blood that might interfere with light transmission, and the light source element is activated. Flushing fluid can be removed, along with any debris. Normal blood flow is allowed to resume for a period of time, and if required, additional light therapy is administered. The first intra lumen device can then be repositioned to treat other portions of the body lumen, if required. For example, in some cases, the light source element cannot illuminate all of the portion of the body lumen isolated by the guide catheter balloon and the distal treatment device, without being repositioned. A similar embodiment of the first intra lumen device includes a balloon disposed proximal of the light source element (or proximal of the hollow shaft adapted to accommodate a separate light source element), so that the guide catheter is not required to include a balloon.

Another embodiment of the present invention includes integrated distal protection devices. In one such embodiment, an outer guide catheter has an occlusion member (such as a balloon) disposed at its distal end, and an inner light emitting catheter. The light emitting catheter includes at least one of a light source element and a substantially transparent hollow shaft, and a hollow tip at its distal end (such that the light emitting catheter can be advanced over a guidewire without requiring a guidewire lumen to be included in the light element portion), as described above. The light emitting catheter further includes a generally light transmissive expandable member substantially encompassing the light source element (or the hollow shaft), so that the light source element can be centered within a body lumen, and so that the expandable member can displace blood that would otherwise block light from reaching the walls of the body lumen (and the target tissue) where the device is disposed. This embodiment further includes a distal protection device formed of a shape memory material that is disposed distal of the light source element The distal protection device is activated by applying thermal energy to the shape memory material. Either a separate heating element is included, or the shape memory material overlays a portion of the light source element, so heat emitted by the light source element increases the temperature of the shape memory material, causing the distal protection device to deploy.

To use this embodiment of an intra lumen device, the guide catheter is positioned proximal of the treatment site, and the light emitting catheter is positioned so that the distal protection device is distal of the treatment site, and the light source element is disposed adjacent to the treatment site. The occlusion member is inflated, and the distal protection device is deployed, thus isolating a portion of the body lumen into which the device is deployed. The expandable member encompassing the light source element is expanded to perform angioplasty (if desired). Flushing fluid is introduced to remove debris, as discussed above. The expandable member is expanded once again, to displace blood that would interfere with light transmission, and the light source element is energized. Flushing fluid is introduced to remove any additional debris. Normal blood flow is allowed to resume for a period of time, and if required, additional light therapy is administered. The light emitting catheter can then be repositioned to treat other portions of the body lumen, if required.

Yet another embodiment of an intra lumen device that includes a distal protection device has a first and a second generally toroidal inflatable member (i.e., balloons) disposed at a distal end of the intra lumen device. An impermeable sleeve extends between the two inflatable members, forming a conduit within the sleeve through which blood (or other bodily fluid) is diverted when the inflatable members are inflated. Inflating the inflatable members results in a portion of a body lumen in which the device is disposed being isolated, without interrupting blood flow in the body lumen. The portion of the intra lumen device within the sleeve includes a light source element (or the hollow shaft adapted to accommodate a separate light source element). A light transmissive expandable member encompasses the light source element, as noted above. The intra lumen device includes a flushing lumen adapted to introduce (and remove) flushing fluid in the isolated portion of the body lumen (that portion between the inflatable members and the sleeve). It will be appreciated that the distal most inflatable member functions as a distal protection device. Preferably, the light source element is movable relative to the inflatable members, so that the light source element can be repositioned without deflating and re-inflating the inflatable members.

To use this intra lumen device, it is positioned within a body lumen so that a treatment area is disposed between the two inflatable members. The light source element is disposed adjacent the treatment site. The inflatable members are inflated, and the expandable member encompassing the light source element is expanded initially to perform angioplasty, if desired (note that the sleeve must be sufficiently flexible to accommodate this function). Flushing fluid is introduced to remove debris, as indicated above, and to keep the isolated portion free of blood that would interfere with light transmission. The expandable member is expanded once again, sufficiently to occlude blood flow within the sleeve (since the blood flow would interfere with light transmission), and the light source element is energized. Preferably, blood flow is occluded for less than about 50 seconds. Normal blood flow is allowed to resume for a period of time (preferably about 50 seconds), and if required, additional light therapy is administered. Flushing within the isolated portion is continued as needed to remove debris. The light source element can then be repositioned to treat other portions of the body lumen, as required.

### Brief Description of the Drawing Figures

The foregoing aspects and many of the attendant advantages of this invention will become more readily appreciated as the same becomes better understood by reference to the following detailed description, when taken in conjunction with the accompanying drawings, wherein:
FIGURE 1A schematically illustrates a first embodiment of a light-generating device for use with a distal protection device during an intervention;
FIGURE 1B schematically illustrates a guide catheter and a distal protection device being deployed in a vessel during an intervention;
FIGURE 1C schematically illustrates the light-generating device of FIGURE 1A, the guide catheter of FIGURE 1B, and the distal protection device of FIGURE 1B being used together during an intervention;
FIGURE 1D is a cross-sectional view of the light-generating device of FIGURE 1A;
FIGURE 2A schematically illustrates a second embodiment of a light-generating device for use with a distal protection device during an intervention;
FIGURE 2B schematically illustrates a guide catheter and a distal protection device being deployed in a vessel during an intervention;
FIGURE 2C schematically illustrates the light-generating device of FIGURE 2A, the guide catheter of FIGURE 2B, and the distal protection device of FIGURE 2B being used together during an intervention;
FIGURE 2D is a cross-sectional view of the light-generating device of FIGURE 2A;
FIGURE 3A schematically illustrates a heart, indicating the position of a saphenous vein graft;
FIGURE 3B schematically illustrates a light-generating device with a distal protection device being used for an intervention;
FIGURES 3C and'3D are cross-sectional views of the light-generating device of FIGURE 3B;
FIGURE 3E schematically illustrates an embodiment of a light-generating device that is based on the light-generating device of FIGURE 3B, in which heat from the light-generating device is used to deploy a shape memory material comprising the distal protection device;
FIGURE 3F schematically illustrates a light-generating device based on the light-generating device of FIGURE 3B, in which heat from a heating element is used to deploy the shape memory material comprising the distal protection device;
FIGURE 4A schematically illustrates another implementation of a light-generating device with a distal protection device, during an intervention;
FIGURE 4B is a cross-sectional view of the light-generating device of FIGURE 4A; and
FIGURE 4C is an enlarged view of a portion of FIGURE 4A.

### Description of the Preferred Embodiment

Unless otherwise defined, it should be understood that each technical and scientific term used herein and in the claims that follow is intended to be interpreted in a manner consistent with the meaning of that term as it would be understood by one of skill in the art to which this invention pertains. In the event that more than one definition is provided herein, the explicitly defined definition controls.

Various embodiments of light-generating devices that either incorporate distal protection devices, or are adapted to be used with a distal protection device, are described herein. An objective of administering PDT with the present invention may be either diagnostic, wherein the wavelength or waveband of the light being produced is selected to cause the photoreactive agent to fluoresce, thus yielding information about a target tissue, or therapeutic, wherein the wavelength or waveband of the light delivered to photosensitized tissue under treatment causes the photoreactive agent to undergo a photochemical interaction in the tissue that yields free radical species, such as singlet oxygen, causing the photosensitized tissue lysing or destruction.

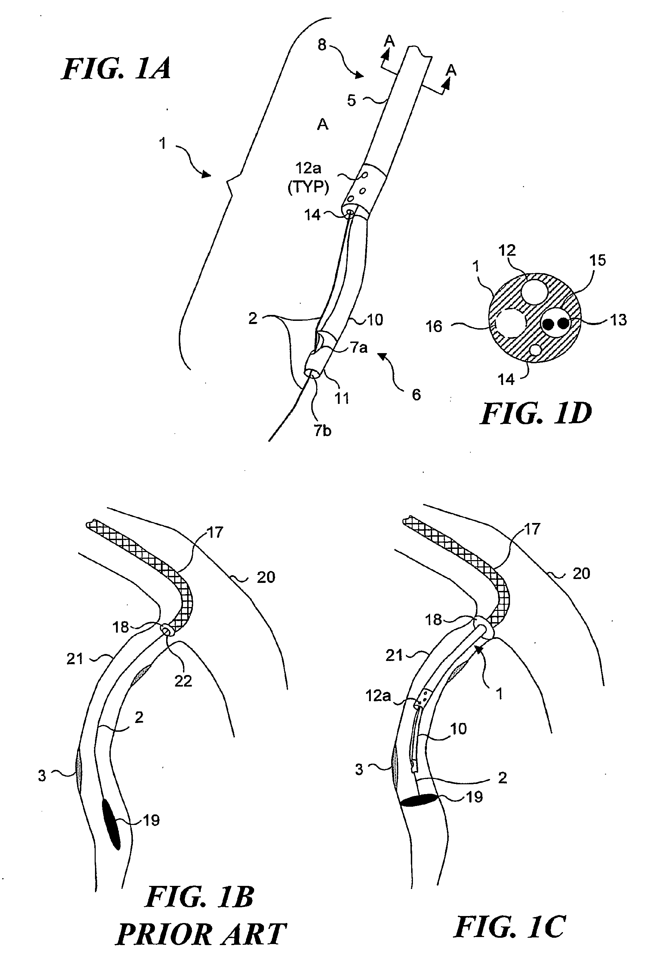

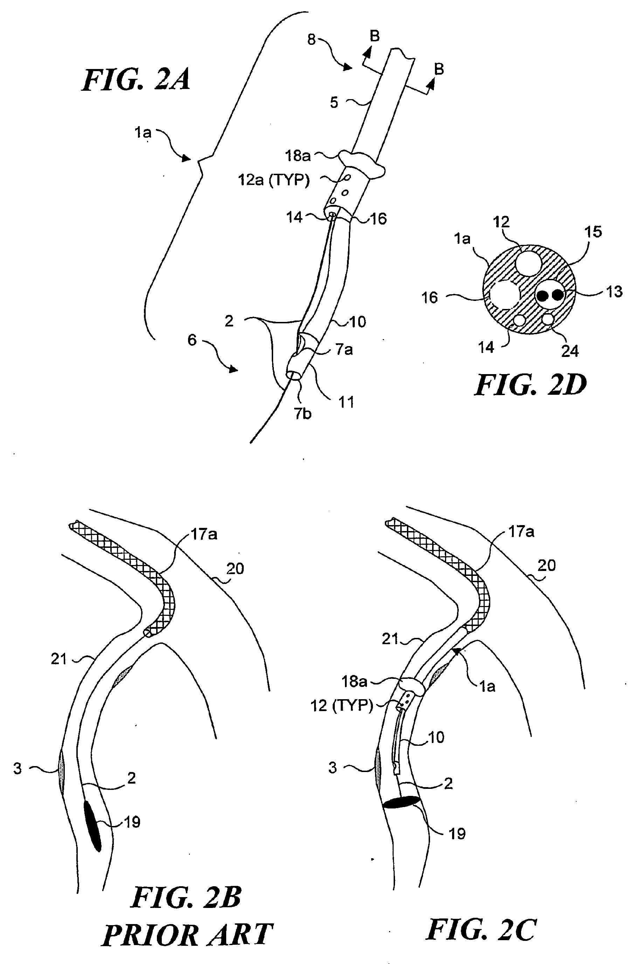

Referring to FIGURE 1A, a light-generating device 1 comprises a multi-lumen catheter having an elongate flexible body 5, formed from a suitable biocompatible material, such as a polymer or metal. Light generating device 1 is adapted to be used with prior art distal protection devices, as explained in greater detail below, and includes a distal end 6, a proximal end 8 (normally disposed outside a body lumen and configured to enable light-generating device 1 to be manipulated), a flushing lumen 12, a guidewire lumen 14, an optional working lumen 16, an optional power lumen 15, and a light source array 10 (see FIGURE 1D, which shows lumens 12, 14, 15, and 16). Generally, either a working lumen or a power lumen will be included, as discussed in detail below. The relative configuration of the lumens as shown in FIGURE 1D is intended to be exemplary, and other configurations can be employed in the alternative. Thus, the relative orientations of the lumens of FIGURE 1D is not intended to be limiting of the present invention. Furthermore, the lumens shown in FIGURE 1D are not drawn to scale, and the relative sizes of the lumens shown are exemplary, rather than controlling. These comments, which specifically pertain to the cross sectional view of FIGURE 1D, also apply to the cross sectional views of FIGURES 2D, 3C, 3D, and 4B.

Guidewire lumen 14 enables elongate flexible body 5 to be advanced over a guidewire, and flushing lumen 12 enables a flushing fluid to be introduced into a body lumen proximate distal end 6 of elongate flexible body 5. Guidewire lumen 14 comprises a hollow conduit of a diameter sufficient to accommodate a guidewire therein and extends between distal end 6 and proximal end 8. As indicated in FIGURE 1A, the guidewire is disposed externally of light-generating device 1 near a light source array 10, so that light source array 10 is not required to include a guidewire lumen.

As can be seen from Figure 1A, the light emitting portion 10 has a smaller diameter than elongate flexible body 5. Further, the light emitting portion 10 does not block the opening 14 through which the guidewire 2 exits the distal end to the elongate flexible body. Flushing lumen 12 is preferably used to convey saline, or another appropriate fluid (such as heparin, a light scattering medium such as Intralipid, or an optically clear, biocompatible fluid), to displace bodily fluids (such as blood) proximate distal end 6, when light-generating device 1 is disposed in a body lumen. Such bodily fluids (especially blood) undesirably interfere with the transmission of light from light source array 1 to target tissue. The flushing fluid is introduced into the body lumen via ports 12a that are disposed on distal end 6 of elongate flexible body 5, proximate light source array 10.

Light source array 10 includes one or more LEDs coupled to conductive traces 13 that are electrically connected to leads extending proximally through a power lumen 15 of light-generating device 1, to an external power supply and control device (not shown). As an alternative to LEDs, other sources of light maybe used, such as organic LEDs, superluminescent diodes, laser diodes, fluorescent light sources, incandescent sources, and light emitting polymers. Light source array 10 is preferably encapsulated in silicone, or another biocompatible polymer, and is coupled to the distal end of elongate flexible body 5.

Optional working lumen 16 is configured to enable an non integrated light source array to be employed. Instead of including integrated light source array 10, light-generating device 1 can be configured without any integrated light source, so that a separate light source array is advanced to the target area through the working lumen after light-generating device 1 is properly positioned in the body lumen. Of course, if a non integral light source array is used, power lumen 15 is not necessary (the power leads for the separate light source element being disposed in the working lumen) and may thus be omitted. If a separate light source array is used, then a hollow, light transmissive shaft is disposed between tip 11 and ports 12a (i.e., if a separate light source array is employed, then reference numeral 10 corresponds to a hollow, light transmissive shaft configured to accommodate a light source array).

Distal end 6 of light-generating device 1 includes a hollow tip 11 coupled to a distal end of light source array 10, (or to the hollow shaft if used in place of light source array 10), with an outwardly facing orifice 7a, as well as a distal orifice 7b, which enable light-generating device 1 to be advanced over a guidewire 2. Note that guidewire lumen 14 does not extend into light source array 10, and thus, the guidewire is disposed external to proximate light source array 10. To position light-generating device 1 in a body lumen, a guidewire 2 is introduced into an artery (or other body lumen) and advanced until the guidewire is disposed adjacent a treatment area (generally an arterial lesion). Elongate flexible body 5 is then advanced over guidewire 2, until distal end 6 is adjacent to the treatment area.

FIGURE 1B schematically illustrates a prior art distal protection device 19, such as a PERCUSURGE™ or a ANGIOGUARD™ Filter, that has been advanced through a guide catheter 17, through aorta 20, for placement at an anastomosis of a saphenous vein graft 21. Guide catheter 17 includes an occlusion balloon 18 disposed near a distal end 22 of guide catheter 17. As shown in FIGURE 1C, light generating device 1 is advanced into saphenous vein graft 21, until it is disposed between balloon 18 and distal protection device 19. The guide catheter includes a working lumen that is larger than light-generating device 1, so that light-generating device 1 is advanced to the treatment site within the working lumen of the guide catheter.

Once light-generating device 1 is properly positioned, occlusion balloon 18 is inflated to block blood flow. Saline solution (or an another biocompatible solution that facilitates light transmission) is flushed through flushing lumen 12 of light generating device 1 to displace the blood in saphenous vein graft 21, thereby facilitating light illumination of target tissue 3. Distal protection device 19 is activated (i.e., expanded), and light-generating device 1 is energized to illuminate target tissue 3. Target tissue 3 will preferably have previously been treated with a photoreactive agent, but if the particular photoreactive agent employed is rapidly taken up by target tissue 3, light generating device 1 can be used to deliver the photoreactive agent through flushing lumen 12, or through a dedicated drug delivery lumen (not shown).

Distal protection device 19 is used to capture atherosclerotic debris that may be generated during the treatment of target tissue 3. Such debris, if allowed to escape downstream, may result in clinically significant and undesirable events, including myocardial infarction, stroke, and renal failure. As noted above, studies have shown that the use of distal protection devices reduces the incidence of major adverse cardiac events by as much as 50 percent. Light generating device 1 can be moved within saphenous vein graft 21 to enable the light source array to illuminate other target tissue, if the target area extends beyond an area that can be illuminated at one time.

FIGURES 2A-2D schematically illustrate a related embodiment of a light generating device 1a, which is intended to be used in a fashion similar to that described above, in regard to light-generating device 1. The difference between light generating device 1 (FIGURES 1A, 1C, and 1D) and light generating device 1a (FIGURES 2A, 2C, and 2D) is that light generating device 1a includes a low-pressure compliant occlusion balloon 18a, and an inflation lumen 24 (see FIGURE 2D). Accordingly, a guide catheter 17a (see FIGURES 2B and 2C) is not required to include an occlusion balloon, as is necessary for guide catheter 17 of FIGURES 1B and 1C. Because the guide catheter is not required to include a balloon, it is possible, but less preferred, for the guide catheter to be smaller than optional working lumen 16 of light-generating device 1a, so that light-generating device 1a is advanced to the treatment site over the guide catheter.

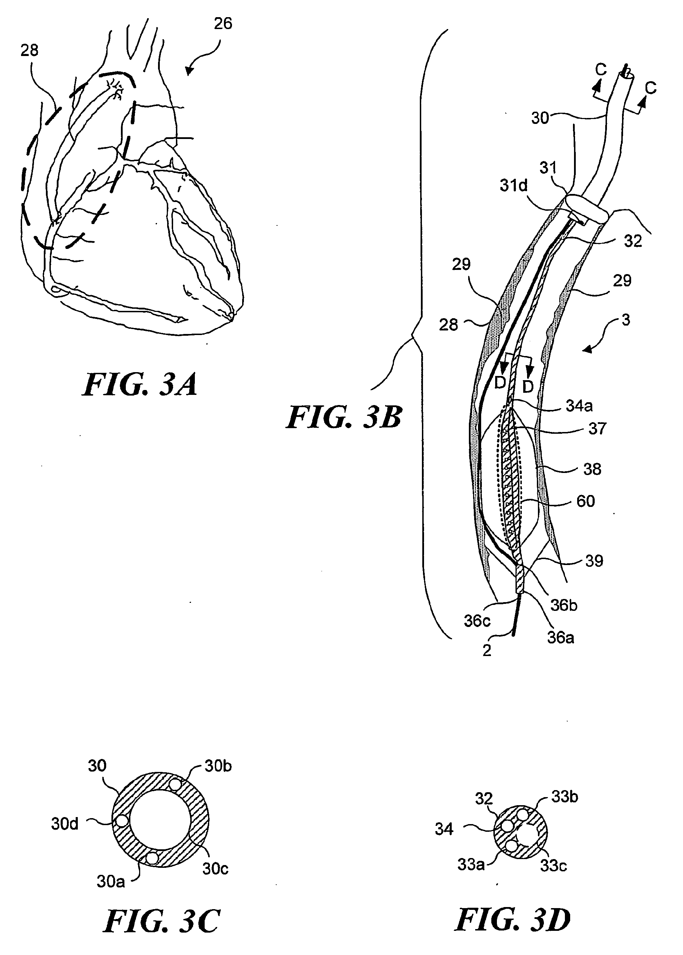

FIGURE 3A schematically illustrates a heart 26, generally indicating the position of a saphenous vein graft 28, a portion of which is depicted in greater detail in FIGURE 3B. The portion of saphenous vein graft 28 shown in FIGURE 3B includes treatment areas 29 (typically having lesions or plaque). Yet another embodiment of a light-generating device is shown in FIGURE 3B. Note that while light generating device 1 of FIGURE 1A-1D, and light generating device 1a of FIGURES 2A-2D each are intended to be used with a prior art distal protection device, the light generating devices discussed in connection with FIGURES 3A-3F include a distal protection member. Referring to FIGURE 3B, a light-generating device 3 includes a guiding catheter 30 and a multi-lumen light-generating catheter 32. Guiding catheter 30 includes a low pressure occlusion balloon 31 (disposed near the distal end of guiding catheter 30). Also, as shown in FIGURE 3C, guiding catheter 30 has a guidewire lumen 30a, an inflation lumen 30b (adapted to enable low pressure occlusion balloon 31 to be selectively inflated), a working lumen 30c, and an aspiration lumen 30d. Working lumen 30c is configured to accommodate light-generating catheter 32, so that light-generating catheter 32 can be advanced to a treatment site within the working lumen of guide catheter 30. Aspiration lumen 30d enables a flushing fluid introduced via light-generating catheter 32 (described in detail below) to be removed from the body lumen. However, if desired, the flushing lumen in light generating catheter 32 can be used both to introduce a flushing fluid, and to aspirate the flushing fluid previously introduced, so that aspiration lumen 30d is then not required.

Light-generating catheter 32 has an elongate flexible body formed from a suitable biocompatible material, such as a polymer or metal. As shown in FIGURE 3D, light-generating catheter 32 also has a plurality of lumens, including a flushing lumen 34, a guidewire lumen 33a, an inflation lumen 33b, and an optional working lumen 33c.

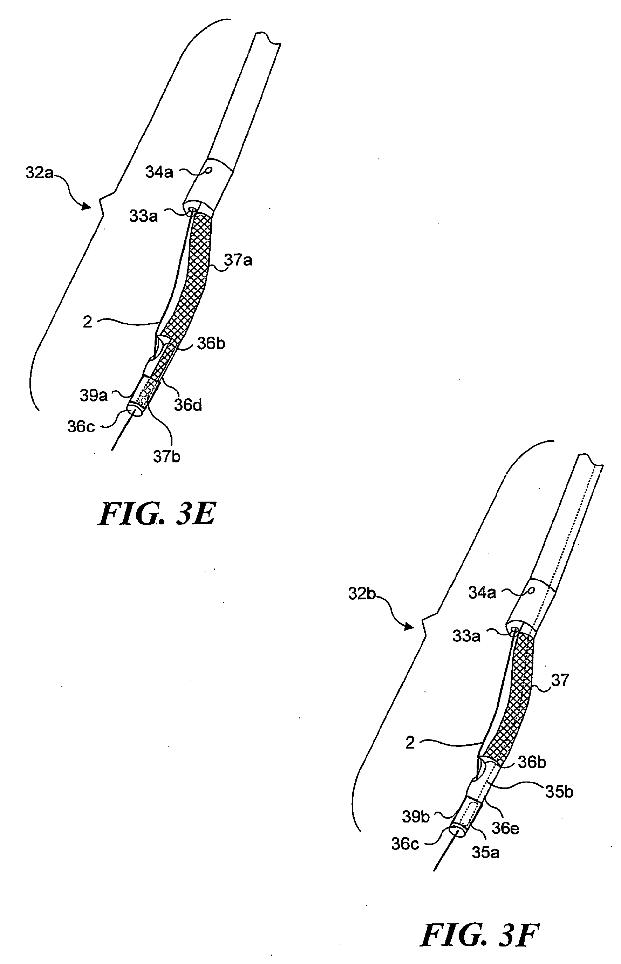

Referring back to FIGURE 3B, flushing medium is introduced into a body lumen into which light-generating catheter 32 is disposed through one or more ports 34a. Once again, the flushing medium may be saline solution or any other appropriate medium that is suitable to displace the bodily fluids (such as blood) in a body lumen, to facilitate light illumination of the target tissue. Guidewire lumen 33a is a hollow conduit of a diameter sufficient to accommodate a guidewire therein, and extends between a distal end of light-generating catheter 32 and a proximal end of light-generating catheter 32. As indicated in FIGURE 3, the guidewire is disposed externally of light-generating catheter 32 near a light emitting portion, so that the light emitting portion is not required to include a guidewire lumen. The distal end of light-generating catheter 32 includes a hollow tip 36a with an orifice 36b that faces toward a wall of the body lumen, and a distal orifice 36c (in a configuration similar to that shown in FIGURE 1A for light generating device 1). Orifices 36b and 36c facilitate the advancement of light-generating catheter 32 over guidewire 2.

Light-generating catheter 32 includes a light source array 37, which can optionally be coupled to collection optics (not shown). As discussed above in connection with light source array 10 of FIGURE 1A, light source array 37 may include one or more LEDs coupled to conductive traces that are electrically connected to leads extending proximally through a lumen of the light generating catheter to an external power supply and control device (not shown). As an alternative to LEDs, other sources of light maybe used, such as organic LEDs, superluminescent diodes, laser diodes, fluorescent light sources, incandescent sources, and light emitting polymers. Light source array 37 is preferably encapsulated or otherwise covered with a substantially optically transparent (at least with regard to the wavelengths emitted by light source array 37) biocompatible polymer, such as silicone. Light source array 37 can be integral to light-generating catheter 32 (in which case, light-generating catheter 32 preferably includes a power lumen to convey the electrical leads that are employed to couple the light source array to an external power supply), or light source array 37 can be a separate component that is advanced to the treatment site using optional working lumen 33c, after light-generating catheter 32 is properly positioned in the body lumen. If light source array 37 is a separate component, then light-generating catheter 32 includes a transparent hollow shaft 60, adapted to accommodate light source array 37 (such a shaft is also described above, in connection with FIGURE 1A and light generating device 1).

Light-generating catheter 32 also includes an expandable member 38, for centering the distal end of light-generating catheter 32, and for either occluding blood flow or for performing angioplasty (or both). Inflation lumen 34 is adapted to selectively control the inflation of expandable member 38, which is preferably secured to the distal portion of light-generating catheter 32 so as to encompass light source array 37. Expandable member 38 comprises a suitable biocompatible material, such as, polyurethane, polyethylene, fluorinated ethylene propylene (FEP), polytetrafluoroethylene (PTFE) or PET (polyethylene terephthalate), and preferably, is substantially light transmissive, since light from light source array 37 must freely pass through expandable member 38 to reach the target tissue. Proximal of expandable member 38 and orifice 36b is a shape memory filter 39 that traps and removes emboli and/or other debris from the body lumen within which light-generating catheter 32 is being used.

Shape memory filter 39 moves between its first and second positions in response to a temperature change, preferably, an increase in temperature. An application of heat increases the temperature of the shape memory material above its transition temperature. The shape memory material memorizes a certain shape at a certain temperature and can be selectively activated to return to its memorized shape by applying heat to the shape memory material so that it is heated above the transition temperature. Preferably the shape memory material is a polymer; such shape memory materials are well known in the art and need not be described herein in detail. The first position of shape memory filter 39 corresponds to an un-deployed configuration, wherein shape memory filter 39 generally conforms to the distal end of the light-generating catheter 32. The second position of shape memory filter 39 corresponds to a deployed configuration, wherein shape memory filter 39 generally expands outwardly and away from light-generating catheter 32, until the shape memory filter contacts the walls of the body lumen in which light-generating catheter 32 is deployed, thereby preventing debris from moving past shape memory filter 39.

When light generating device 3 is in use, guiding catheter 30 is introduced into a body lumen and positioned proximal of a treatment area. Then, light-generating catheter 32 is advanced through guiding catheter 30 (and over guidewire 2, distal of guiding catheter 30) until light generating array 37 is disposed adjacent the treatment area. While the light-generating catheter 32 is being advanced over the guidewire to a treatment site, shape memory filter 39 is not deployed. When light-generating catheter 32 is positioned adjacent to the treatment site, shape memory filter 39 is deployed into its second position. Occlusion balloon 31 is inflated, and expandable member 38 is inflated and deflated to perform angioplasty (if desired).

Saline solution is then introduced to the isolated portion of the body lumen (i.e., to the portion between occlusion balloon 31 and shape memory filter 39) via flushing lumen 34 and removed via aspiration lumen 30d. As noted above, flushing and aspiration could be carried out using a single lumen, by first flushing and then aspirating through the lumen. The use of a separate flushing lumen and a separate aspiration lumen enable a circulating flow to be achieved, so that more debris can be removed in a shorter time. Flushing not only removes debris, which might get past shape memory filter 39 as light generating catheter 32 is removed, but also maintains a clear light transmission path to the body lumen wall, keeping the portion of the body lumen between balloon 31 and shape memory filter 39 essentially free of blood and debris. Expandable member 38 is then again inflated to facilitate the transmission of light from light source array 37 to the body lumen wall. Preferably, light source array 37 is rotated within catheter 32, to enable all portions of the lumen walls around the light source array to be illuminated. Alternatively, the light source array can include light sources disposed so that light is emitted outwardly of the light source array through substantially a full 360 degrees of arc.

As noted above, shape memory filter 39 is preferably deployed by using heat. FIGURES 3E and 3F schematically illustrate different embodiments for applying the required thermal energy to shape memory filter 39. Each of FIGURES 3E and 3F includes a light generating catheter substantially similar to light generating catheter 32, except for the modification discussed in detail below to enable shape memory filter 39 to be heated by the light source. In each of FIGURES 3E and 3F, expandable member 38 has been omitted, to reduce the complexity of those Figures.

FIGURE 3E schematically illustrates a light generating catheter 32a (with the expanding member not shown, as noted above). A light source array 37a extends into a hollow tip 36d. A shape memory filter 39a is disposed distal of orifice 36b, to ensure that the guidewire does not interfere with the filter when it is in the deployed position. In FIGURE 3E, shape memory filter 39a is not yet deployed, and part of shape memory filter 39a overlays a portion 37b of light source array 37a. Energizing light source array 37a produces heat that is absorbed by shape memory filter 39a, causing the filter to deploy.

FIGURE 3F illustrates a related embodiment, in which a heater, rather than the light-generating array, is used to provide the heat that changes the temperature of the shape memory material comprising the filter (the distal protection device). In FIGURE 3F, a light-generating catheter 32b is shown. A hollow tip 36e includes orifice 36b, orifice 36c, and a heating element 35a. A shape memory filter 39b is disposed distal of orifice 36b, again to ensure that the guidewire does not interfere with the filter when it is in the deployed position. Shape memory filter 39b does not overlie light source array 37 in light-generating catheter 32b. Instead, shape memory filter 39b is disposed adjacent to heating element 35a, so that the heat produced by energizing heating element 35a causes shape memory filter 39b to deploy. Electrical lead 35b couples heating element 35a to an external power source (not shown). Preferably, heating element 35a is a resistive heating element, such as a nichrome wire, although other types of heating elements can alternatively be employed.

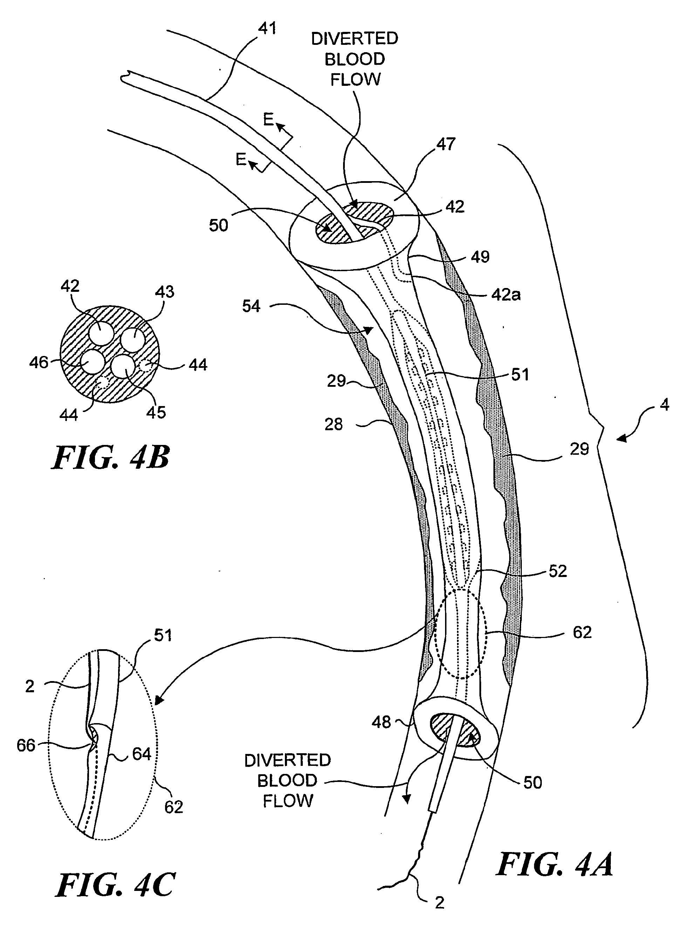

FIGURE 4A schematically illustrates another implementation of a light-generating device with an integrated distal protection device, for use during an interventional procedure. Light-generating device 4, shown disposed in saphenous vein graft 28 (which includes treatment areas 29) comprises a multi-lumen catheter 41 having an elongate, flexible body formed from a suitable biocompatible material, such as a polymer or metal. Catheter 41 includes a proximal torus-shaped protection balloon 47, and a distal torus-shaped protection balloon 48, coupled with an impermeable exclusion sleeve 49 that extends between balloon 47 and balloon 48; sleeve 49 thus defmes a conduit 50. When catheter 41 is disposed in saphenous vein graft 28 (or in another body lumen) and balloons 47 and 48 are inflated, a portion 54 of saphenous vein graft 28 is defined by the walls of saphenous vein graft 28, sleeve 49, and balloons 47 and 48. Portion 54 is isolated from blood flow, which is diverted around portion 54 through conduit 50, thereby excluding treatment areas 29 (i.e., the lesions) from the vascular lumen, and allowing blood flow to continue during the intervention, which prevents embolization. When inflated, balloons 47 and 48 tend to center the portion of catheter 41 extending between the balloons within the body lumen in which the body lumen catheter 41 is deployed.

Catheter 41 also includes a light source array 51, which is generally consistent with the light source arrays described above. Once again, light source array 51 can be an integral part of catheter 41, or the light source array can be a separate component advanced through a working lumen after catheter 41 is properly positioned, as discussed above. Again, if the light source array is not an integral component of catheter, then catheter 41 includes a transparent hollow shaft adapted to accommodate the separate light source array, which is introduced into the hollow shaft via a working lumen, also as described above.

Catheter 41 preferably includes an expandable member 52 that is adapted to occlude blood flow through conduit 50 and to perform angioplasty (if desired). Preferably, expandable member 52 encompasses light-source array 51 (or the hollow shaft adapted to receive the light source array), and is formed from a suitable biocompatible material, such as, polyurethane, polyethylene, FEP, PTFE or PET. Because expandable member 52 encompasses light source array 51, the expandable member is formed of a light transmissive material, so that light from light source array can freely pass through the expandable member to reach the target tissue.

As shown in FIGURE 4A, light source array 51 and expandable member 52 are disposed within conduit 50, so that sleeve 49 (which defines conduit 50) must also be sufficiently transparent so that light from light source array 51 can freely pass through sleeve 49 to reach target tissue 29. Further, where expandable member 52 is intended to be used to perform angioplasty, sleeve 49 must be sufficiently large and flexible, to accommodate expandable member 52 in its fully expanded state (i.e., when expandable member 52 is inflated to contact the walls of the body lumen in which catheter 41 is disposed). If it is not necessary to perform angioplasty, expandable member 52 is inflated only enough to securely position the light source array within sleeve 49. Preferably, sleeve 49 comprises a polymeric material that transmits light of the wavelength or waveband used for the PDT. Preferably, light source array 51 rotatable within catheter 41, to enable all portions of the lumen walls to be illuminated. Alternatively, the light source array can include light sources disposed so that light is emitted outwardly from the light source array through substantially a full 360 degrees of arc, to fully illuminate the treatment area.

FIGURE 4B illustrates the plurality of lumens included in catheter 41, include a flushing and aspiration lumen 42, an inflation lumen 43, which enables expandable member 52 to be selectively inflated and deflated, optional conductive lumens 44, which accommodate a laser fiber or light emitting diode wire, neither of which are shown, but which can be used in addition to or in place of light source array 51, a balloon inflation lumen 45, which enables balloons 47 and 48 to be selectively inflated and deflated (an additional balloon inflation lumen can be incorporated if it is desired to independently control the inflation/deflation of balloons 47 and 48), and a guidewire lumen 46, which accommodates guidewire 2, to enable catheter 41 to be advanced over the pre-positioned guidewire. Flushing and aspiration lumen 42 is connected to lumen portion 54 through one or more ports 42a that pass through sleeve 49. As described above, the flushing fluid is used to displace blood and debris in portion 54, and to facilitate illumination of target tissue 29 using light source array 51. Exemplary suitable flushing fluids include saline solution, and the other flushing fluids noted above. While a working lumen to accommodate a separate light source array is not specifically shown, it should be understood that such a working lumen is readily included in catheter 41 (such working lumens have been indicated in FIGURES 1D, 2D, and 3D).

To use catheter 41, guidewire 2 is first introduced into the body lumen to be treated and advanced to just beyond the target tissue. Catheter 41 is then advanced into the body lumen over guidewire 2, until light source array 51 (or the hollow shaft adapted to receive the light source array) is disposed adjacent to target tissue 29. Torus-shaped balloons 47 and 48 are then inflated, isolating the portion of the lumen between the balloons. Blood continues to flow through conduit 50. Expandable member 52 is inflated to perform angioplasty (if desired). Saline solution is then flushed and aspirated through flushing and aspiration lumen 42 to maintain a clear light transmission path to the vessel wall essentially free of blood and debris. Expandable member 52 is again inflated, to displace blood flowing within conduit 50, which may interfere with the transmission of light from light source array 51, and to securely position the light source array within sleeve 49. Light source array 51 is energized, preferably for less than about 50 seconds. During the administration of light to the target tissue, expandable member 52 occludes blood flow in conduit 50. It is believed that interrupting blood flow for less than about 50 seconds, followed by enabling blood flow to resume for about 50 seconds (to enable the blood to re-perfuse), should obviate problems that are sometimes encountered when blood flow is occluded for longer intervals. Thus, expandable member 52 can be expanded and deflated cyclically, for periods of about 50 seconds each, to administer the desired PDT to a specific target area. Portion 54 (partially defined by balloons 47 and 48) may extend beyond the illumination limits of light source array 51. Preferably, the light source array is then selectively repositioned within portion 54, without having to move balloons 47 and 48, to enable the light source array to administer PDT to all target tissue in portion 54.

One structure that enables light source array 51 to be selectively repositioned without moving balloons 47 and 48 is achieved by forming the catheter body between the balloons from a substantially light transmissive polymer material. Light source array 51 is then slidably disposed in a working lumen in the catheter body, so that the light source array can be repositioned as desired. Such working lumens are shown in FIGURES 1D, 2D and 3D. Expandable member 52 is coupled to the light transmissive portion of the catheter body (i.e., the portion of catheter 41 encompassed by sleeve 49), so that blood flow through sleeve 49 can be occluded when the light source array is energized.

FIGURE 4C illustrates that catheter 41 preferably includes a hollow tip 64, which is disposed distally of light source array 51 and proximally of balloon 48. Hollow tip 64 includes a side facing orifice 66 that enables catheter 41 to be advanced over guidewire 2 (i.e., light source array 51 does not include a guidewire lumen, and the guidewire is exposed externally to catheter 41, proximate light source array 51). This configuration is shown in greater detail in FIGURES 1A, 2A, 3E, and 3F). Alternatively, but not separately shown, light source array 51 includes a guidewire lumen, or guidewire 2 can be withdrawn once balloons 47 and 48 are inflated, so that a separate light source array can be advanced through the guidewire lumen. Balloon 52 has been omitted from FIGURE 4C, to simplify the Figure.

Although the present invention has been described in connection with the preferred form of practicing it and modifications thereto, those of ordinary skill in the art will understand that many other modifications can be made to the present invention within the scope of the claims that follow. Accordingly, it is not intended that the scope of the invention in any way be limited by the above description, but instead be determined entirely by reference to the claims that follow.

## Claims

1. Apparatus for illuminating a portion of a body lumen (21, 28), comprising:
(a) an elongate flexible body (5, 30, 41) having a proximal end, a distal end, and a plurality of lumens, said plurality of lumens including at least a guidewire lumen (14, 30a, 46) and a flushing fluid lumen (12, 30d, 42), the guidewire lumen not extending beyond the distal end of the elongate flexible body (5, 30, 41), the distal end of the elongate flexible body (5, 30, 41) including an opening for accessing the guidewire lumen (14, 30a, 46), the guidewire lumen opening enabling a guidewire to extend beyond the distal end of the elongate flexible body (5, 30, 41);
(b) a light emitting portion (10, 32, 37, 37a, 51) disposed distal of the distal end of the elongate flexible body (5, 30, 41), wherein the light emitting portion (10, 32, 37, 37a, 51) comprises a light source (37, 51) that emits light having a characteristic emission waveband selected for administering photodynamic therapy to the body lumen (21, 28), the light emitting portion (10, 32, 37, 37a, 51) having a smaller diameter than the elongate flexible body (5, 30, 41), such that the light emitting portion (10, 32, 37, 37a, 51) does not block the guidewire lumen opening at the distal end of the elongate flexible body (5, 30, 41); and
(c) a hollow tip (11, 36a, 64) disposed distal of the light emitting portion (10, 32, 37, 37a, 51), the hollow tip (11, 36a, 64) having a distal face including a first orifice (7b, 36c), and a side surface including a second orifice (7a, 36b, 66), the first and the second orifices enabling the apparatus to be advanced over a guidewire without requiring the light emitting portion to include the guidewire lumen.

2. The apparatus of Claim 1, wherein the light emitting portion (10, 32, 37, 37a, 51) comprises an array of light sources (10, 37, 37a, 51).

3. The apparatus of Claim 1, wherein a portion of a guidewire that is proximate to the light source (10, 32, 37, 37a, 51) is exposed to the body lumen (21, 28) before being threaded through the second orifice (7a, 36b, 66) and the first orifice (7b, 36c) of the hollow tip (11, 36a, 64).

4. The apparatus of Claim 2, wherein the array of light sources (10, 37, 37a, 51) comprises a plurality of light emitting diodes.

5. The apparatus of Claim 1, wherein the light emitting portion (10, 32, 37, 37a, 51) comprises an array of light sources (10, 37, 37a, 51), and wherein the array of light sources (10, 37, 37a, 51) is mounted on a flexible, conductive substrate encapsulated in silicone.

6. The apparatus of Claim 1, further comprising an inflatable member (48) disposed proximal of the light emitting portion (51).

7. The apparatus of Claim 1, further comprising a generally light (38, 52) transmissive expandable member encompassing the light emitting portion (37, 51).

8. The apparatus of Claim 1, further comprising a distal protection device (19, 39, 39a, 39b, 48) disposed distal of the light emitting portion (10, 32, 37, 37a, 51), the distal protection device (19, 39, 39a, 39b, 48) being movable between a first position and a second position, the first position being **characterized by** the distal protection device (19, 39, 39a, 39b, 48) generally conforming to a portion (2, 36a, 36d, 36e) of the apparatus to which the distal protection device (19, 39, 39a, 39b, 48) is attached, the second position being **characterized by** the distal protection device (19, 39, 39a, 39b, 48) generally extending from the portion (2, 36a, 36d, 36e) of the apparatus to which the distal protection device (19, 39, 39a, 39b, 48) is attached to a wall of a body lumen (21, 28), so that the distal protection device (19, 39, 39a, 39b, 48) either filters a flow of bodily fluid through the body lumen (21, 28) or substantially occludes a flow of bodily fluid through the body lumen (21, 28), thereby preventing debris from moving past the distal protection device (19, 39, 39a, 39b, 48).

9. The apparatus of Claim 8, wherein the distal protection device (19, 39, 39a, 39b) comprises a shape memory material, the shape memory material moving from the first position to the second position in response to a change in temperature.

10. The apparatus of Claim 9, wherein the distal protection device (39a) overlaps at least part of the light emitting portion (37b), so that heat from the light emitting portion (37b) increases a temperature of the distal protection device (39a), thereby causing the distal protection device (39a) to move to the second position as a result of a force produced by the shape memory material.

11. The apparatus of Claim 9, further comprising a heating element (35a) disposed adjacent to the distal protection device (39b), and an electrical lead having a proximal end adapted to be electrically coupled to an external power supply and a distal end electrically coupled to the heating element (35a), thereby enabling the heating element (35a) to be energized with an electrical current when the proximal end of the electrical lead is electrically coupled to an external power supply, heat produced by the heating element (35a) causing the distal protection device (39b) to move to the second position as a result of a force produced by the shape memory material.

12. The apparatus of Claim 8, wherein the distal protection device (19, 39, 39a, 39b) is disposed distal of the second orifice (7a, 36b, 66) of the hollow tip (11, 36a, 64).

13. The apparatus of Claim 8, further comprising an inflatable member (18a, 31, 47) disposed proximal of the light emitting portion (10, 37, 51), so that when activated, the inflatable member (18a, 31, 47) and the distal protection device (19, 39, 48) isolate a portion of a body lumen (21, 28) in which the apparatus is disposed.

14. The apparatus of Claim 13, wherein the light emitting portion (10, 37) is positionable independently of the inflatable member (18a, 31).

15. The apparatus of Claim 13, wherein the distal protection device (48) comprises an additional inflatable member (47).

16. The apparatus of Claim 15, wherein the distal protection device (48) and the inflatable member (47) are generally toroidal.

17. The apparatus of Claim 15, further comprising a generally light transmissive non porous sleeve (49) extending between the inflatable member (47) and the distal protection device (48), the sleeve (49) defining a conduit through which a flow of bodily fluid is diverted when the apparatus is disposed in a body lumen (28) and the inflatable member (47) and the distal protection device (48) are activated.

18. The apparatus of Claim 17, wherein the light emitting portion (51) is encompassed by the sleeve (49).

19. The apparatus of Claim 17, wherein the light emitting portion (51) is encompassed by a generally light transmissive expandable member (52), the expandable member (52) being encompassed by the sleeve (49), so that when the expandable member (52) is activated, any fluid flow through the conduit defined by the sleeve (49) is occluded.

20. The apparatus of Claim 17, wherein the flushing lumen (42) is coupled to at least one port (42a) in fluid communication with a portion (54) of a body lumen (28) that is isolated when the apparatus is disposed in a body lumen (28) and the inflatable member (47) and the distal protection device (48) are activated.

## Patentansprüche

1. Vorrichtung zum Beleuchten eines Abschnitts eines Körperlumens (21, 28), die umfasst:
a) einen länglichen flexiblen Körper (5, 30, 41) mit einem proximalen Ende, einem distalen Ende und einer Vielzahl von Lumen, wobei die Vielzahl von Lumen wenigstens ein Führungsdraht-Lumen (14, 30a, 46) und ein Spülfluid-Lumen (12, 30d, 42) enthalten, sich das Führungsdraht-Lumen nicht über das distale Ende des länglichen flexiblen Körpers (5, 30, 41) hinaus erstreckt, das distale Ende des länglichen flexiblen Körpers (5, 30, 41) eine Öffnung für Zugang zu dem Führungsdraht-Lumen (14, 30a, 46) enthält und die Öffnung des Führungsdraht-Lumens zulässt, dass sich ein Führungsdraht über das distale Ende des länglichen flexiblen Körpers (5, 30, 41) hinaus erstreckt;
b) einen Lichtemittierabschnitt (10, 32, 37, 37a, 51), der distal zu dem distalen Ende des länglichen flexiblen Körpers (5, 30, 41) angeordnet ist, wobei der Lichtemittierabschnitt (10, 32, 37, 37a, 51) eine Lichtquelle (37, 51) umfasst, die Licht mit einem charakteristischen Emissions-Wellenband emittiert, das zum Durchführen fotodynamischer Therapie des Körperlumens (21, 28) ausgewählt wird, der Lichtemittierabschnitt (10, 32, 37, 37a, 51) einen kleineren Durchmesser hat als der längliche flexible Körper (5, 30, 41), so dass der Lichtemittierabschnitt (10, 32, 37, 37a, 51) die Öffnung des Führungsdraht-Lumens am distalen Ende des länglichen flexiblen Körpers (5, 30, 41) nicht blockiert; und
c) eine hohle Spitze (11, 36a, 64), die distal zu dem Lichtemittierabschnitt (10, 32, 37, 37a, 51) angeordnet ist, wobei die hohle Spitze (11, 36a, 64) eine distale Fläche, die ein erstes Loch (7b, 36c) enthält, und eine seitliche Fläche aufweist, die ein zweites Loch (7a, 36b, 66) enthält, und das erste sowie das zweite Loch zulassen, dass die Vorrichtung über einen Führungsdraht verschoben wird, ohne dass der Lichtemittierabschnitt das Führungsdraht-Lumen enthalten muss.

2. Vorrichtung nach Anspruch 1, wobei der Lichtemittierabschnitt (10, 32, 37, 37a, 51) eine Anordnung von Lichtquellen (10, 37, 37a, 51) umfasst.

3. Vorrichtung nach Anspruch 1, wobei ein Abschnitt eines Führungsdrahtes, der sich nahe an der Lichtquelle (10, 32, 37, 37a, 51) befindet, zu dem Körperlumen (21, 28) hin freigelegt wird, bevor er durch das zweite Loch (7a, 36b, 66) und das erste Loch (7b, 36c) der hohlen Spitze (11, 36a, 64) geführt wird.

4. Vorrichtung nach Anspruch 2, wobei die Anordnung von Lichtquellen (10, 37, 37a, 51) eine Vielzahl von Leuchtdioden umfasst.

5. Vorrichtung nach Anspruch 1, wobei der Lichtemittierabschnitt (10, 32, 37, 37a, 51) eine Anordnung von Lichtquellen (10, 37, 37a, 51) umfasst und die Anordnung von Lichtquellen (10, 37, 37a, 51) auf einem flexiblen leitenden Substrat angebracht ist, das in Silikon gekapselt ist.

6. Vorrichtung nach Anspruch 1, die des Weiteren ein aufblasbares Element (48) umfasst, das proximal zu dem Lichtemittierabschnitt (51) angeordnet ist.

7. Vorrichtung nach Anspruch 1, die des Weiteren ein im Allgemeinen lichtdurchlässiges dehnbares Element (38, 52) umfasst, das den Lichtemittierabschnitt (37, 51) umschließt.

8. Vorrichtung nach Anspruch 1, die des Weiteren eine distale Schutzeinrichtung (19, 39, 39a, 39b, 48) umfasst, die distal zu dem Lichtemittierabschnitt (10, 32, 37, 37a, 51) angeordnet ist, wobei die distale Schutzeinrichtung (19, 39, 39a, 39b, 48) zwischen einer ersten Position und einer zweiten Position bewegt werden kann, die erste Position **dadurch gekennzeichnet ist, dass** sich die distale Schutzeinrichtung (19, 39, 39a, 39b, 48) im Allgemeinen an einen Abschnitt (2, 36a, 36d, 36e) der Vorrichtung anpasst, an dem die distale Schutzeinrichtung (19, 39, 39a, 39b, 48) angebracht ist, und die zweite Position **dadurch gekennzeichnet ist, dass** sich die distale Schutzeinrichtung (19, 39, 39a, 39b, 48) im Allgemeinen von dem Abschnitt (2, 36a, 36d, 36e) der Vorrichtung, an dem die distale Schutzeinrichtung (19, 39, 39a, 39b, 48) angebracht ist, zu einer Wand eines Körperlumens (21, 28) erstreckt, so dass die distale Schutzeinrichtung (19, 39, 39a, 39b, 48) entweder einen Strom von Körperfluid durch das Körperlumen (21, 28) filtert oder im Wesentlichen einen Strom von Körperfluid durch das Körperlumen (21, 28) blockiert und so verhindert, dass sich Verunreinigungen über die distale Schutzeinrichtung (19, 39, 39a, 39b, 48) hinausbewegen.

9. Vorrichtung nach Anspruch 8, wobei die distale Schutzeinrichtung (19, 39, 39a, 39b) ein Formgedächtnis-Material umfasst und sich das Formgedächtnis-Material in Reaktion auf eine Temperaturänderung von der ersten Position an die zweite Position bewegt.

10. Vorrichtung nach Anspruch 9, wobei die distale Schutzeinrichtung (39a) wenigstens einen Teil des Lichtemittierabschnitts (37b) überlappt, so dass Wärme von dem Lichtemittierabschnitt (37b) eine Temperatur der distalen Schutzeinrichtung (39a) erhöht und so bewirkt, dass sich die distale Schutzeinrichtung (39a) aufgrund einer durch das Formgedächtnis-Material erzeugten Kraft an die zweite Position bewegt.

11. Vorrichtung nach Anspruch 9, die des Weiteren ein Heizelement (35a), das an die distale Schutzeinrichtung (39b) angrenzend angeordnet ist, sowie eine elektrische Zuleitung umfasst, die ein proximales Ende, das so eingerichtet ist, dass es elektrisch mit einer externen Stromquelle gekoppelt ist, und ein distales Ende hat, das elektrisch mit dem Heizelement (35a) gekoppelt ist, um so zuzulassen, dass das Heizelement (35a) mit einem elektrischen Strom erregt wird, wenn das proximale Ende der elektrischen Zuleitung elektrisch mit einer externen Stromquelle gekoppelt ist, und durch das Heizelement (35a) erzeugte Wärme bewirkt, dass sich die distale Schutzeinrichtung (39b) aufgrund einer durch das Formgedächtnis-Material erzeugten Kraft an die zweite Position bewegt.

12. Vorrichtung nach Anspruch 8, wobei die distale Schutzeinrichtung (19, 39, 39a, 39b) distal zu dem zweiten Loch (7a, 36b, 66) der hohlen Spitze (11, 36a, 64) angeordnet ist.

13. Vorrichtung nach Anspruch 8, die des Weiteren ein aufblasbares Element (18a, 31, 47) umfasst, das proximal zu dem Lichtemittierabschnitt (10, 37, 51) angeordnet ist, so dass, wenn aktiviert, das aufblasbare Element (18a, 31, 47) und die distale Schutzeinrichtung (19, 39, 48) einen Abschnitt eines Körperlumens (21, 28) isolieren, in dem die Vorrichtung angeordnet ist.

14. Vorrichtung nach Anspruch 13, wobei der Lichtemittierabschnitt (10, 37) unabhängig von dem aufblasbaren Element (18a, 31) positioniert werden kann.

15. Vorrichtung nach Anspruch 13, wobei die distale Schutzeinrichtung (48) ein zusätzliches aufblasbares Element (47) umfasst.

16. Vorrichtung nach Anspruch 15, wobei die distale Schutzeinrichtung (48) und das aufblasbare Element (47) im Allgemeinen ringförmig sind.

17. Vorrichtung nach Anspruch 15, die des Weiteren eine im Allgemeinen lichtdurchlässige nicht poröse Hülle (49) umfasst, die sich zwischen dem aufblasbaren Element (47) und der distalen Schutzeinrichtung (48) erstreckt, wobei die Hülle (49) eine Leitung bildet, über die ein Strom von Körperfluid abgeleitet wird, wenn die Vorrichtung in einem Körperlumen (28) angeordnet ist und das aufblasbare Element (47) sowie die distale Schutzeinrichtung (48) aktiviert sind.

18. Vorrichtung nach Anspruch 17, wobei der Lichtemittierabschnitt (51) von der Hülle (49) umschlossen ist.

19. Vorrichtung nach Anspruch 17, wobei der Lichtemittierabschnitt (51) von einem im Allgemeinen lichtdurchlässigen dehnbaren Element (52) umschlossen ist, das dehnbare Element (52) von der Hülle (49) umschlossen ist, so dass, wenn das dehnbare Element (52) aktiviert wird, jeglicher Fluidstrom durch die von der Hülle (49) gebildete Leitung blockiert wird.

20. Vorrichtung nach Anspruch 17, wobei das Spül-Lumen (42) mit wenigstens einem Anschluss (42a) in Fluidverbindung mit einem Abschnitt (54) eines Körperlumens (28) gekoppelt ist, der isoliert ist, wenn die Vorrichtung in einem Körperlumen (28) angeordnet ist und das aufblasbare Element (47) sowie die distale Schutzeinrichtung (48) aktiviert sind.

## Revendications

1. Appareil pour l'illumination d'une partie d'une lumière corporelle (21, 28), comprenant:
(a) un corps flexible allongé (5, 30, 41) ayant une extrémité proximale, une extrémité distale, et une pluralité de lumières, ladite pluralité de lumières comprenant au moins une lumière fil guide (14, 30a, 46) et une lumière pour un écoulement de fluide (12, 30d, 42), la lumière fil guide ne s'étendant pas au-delà de l'extrémité distale du corps flexible allongé (5, 30, 41), l'extrémité distale du corps flexible allongé (5, 30, 41) comprenant une ouverture pour l'accès à la lumière fil guide (14, 30a, 41), l'ouverture de la lumière fil guide permettant à un fil guide de s'étendre au-delà de l'extrémité distale du corps flexible allongé (5, 30, 41);
(b) une partie émettrice de lumière (10, 32, 37, 37a, 51) disposée de manière distale à l'extrémité distale du corps flexible allongé (5, 30, 41), dans laquelle la partie émettrice de lumière (10, 32, 37, 37a, 51) comporte une source lumineuse (37, 51) émettant de la lumière ayant une longueur d'onde d'émission caractéristique choisie pour l'administration de thérapie photo dynamique à la lumière corporelle (21, 28), la partie émettrice de lumière (10, 32, 37, 37a, 51) ayant un diamètre plus petit que le corps flexible allongé (5, 30, 41), de telle manière que la partie émettrice de lumière (10, 32, 37, 37a, 51) ne bloque pas l'ouverture de la lumière fil guide à l'extrémité distale du corps flexible allongé (5, 30, 41); et
(c) un embout creux (11, 36a, 64) disposé de manière distale par rapport à la partie émettrice de lumière (10, 32, 37, 37a, 51), l'embout creux (11, 36a, 64) ayant une face distale comprenant un premier orifice (7b, 36c), et une surface de côté incluant un second orifice (7a, 36b, 66), les premier et second orifices permettant au dispositif d'être avancé sur le guide sans que la partie émettrice de lumière ne comporte une lumière fil guide.

2. L'appareil selon la revendication 1, dans lequel la partie émettrice de lumière (10, 32, 37, 37a, 51) comporte une zone de sources lumineuses (10, 37, 37a, 51).

3. L'appareil selon la revendication 1, dans lequel une partie du fil guide qui est proche de la source lumineuse (10, 32, 37, 37a, 51) est exposée à la lumière corporelle (21, 28) avant d'être enfilée au travers du second orifice (7a, 36b, 66) et le premier orifice (7b, 36c) de l'embout creux (11, 36a, 64).

4. L'appareil selon la revendication 2, dans lequel la zone de sources lumineuses (10, 37, 37a, 51) comporte une pluralité de diodes LED.

5. L'appareil selon la revendication 1, dans lequel la partie émettrice de lumière (10, 32, 37, 37a, 51) comporte une zone de sources lumineuses (10, 37, 37a, 51), et dans lequel la zone de sources lumineuses (10, 37, 37a, 51) est monté sur un substrat conducteur flexible encapsulé dans du silicium.

6. L'appareil de la revendication 1, comprenant en outre un membre gonflable (48) disposé à proximité de la partie émettrice de lumière (51).

7. L'appareil de la revendication 1, comprenant en outre un membre extensible généralement susceptible de transmettre la lumière, englobant la partie émettrice de lumière (37, 51).

8. L'appareil de la revendication 1, comprenant en outre un dispositif de protection distal (19, 39, 39a, 39b, 48) disposé de manière distale par rapport à la partie émettrice de lumière (10, 32, 37, 37a, 51), le dispositif de protection distale (19, 39, 39a, 39b, 48) étant mobile entre une première position et une seconde position, la première position étant **caractérisée en ce que** le dispositif de protection distale (19, 39, 39a, 39b, 48) se conforme à une partie (2, 36a, 36d, 36e) de l'appareil auquel le dispositif de protection distal (19, 39, 39a, 39b, 48) est fixé, la seconde position étant **caractérisée en ce que** le dispositif de protection distale (19, 39, 39a, 39b, 48) s'étend de manière générale à partir de la partie (2, 36a, 36d, 36e) de l'appareil auquel le dispositif de protection distal (19, 39, 39a, 39b, 48) est fixé à une paroi de la lumière corporelle (18, 28), de telle manière que le dispositif de protection distale (19, 39, 39a, 39b, 48) soit filtre un flux de fluide corporel au travers de la lumière corporelle (21, 28) ou obture de manière significative un flux de fluide corporel au travers de la lumière corporelle (21, 28), empêchant ainsi le déplacement de débris au travers le dispositif de protection distale (19, 39, 39a, 39b, 48).

9. L'appareil de la revendication 8, dans lequel le dispositif de protection distal (19, 39, 39a, 39b) comporte un matériau mémoire formé, le matériau mémoire formé se déplaçant de la première position vers la seconde position suite à un changement de température.

10. L'appareil de la revendication 9, dans lequel le dispositif de protection distal (19) chevauche au moins une partie de la partie émettrice de lumière (37b), de telle manière que la chaleur de la partie de la partie émettrice de lumière (37b) accroît la température du dispositif de protection distal (19), entraînant ainsi le déplacement du dispositif de protection distal (39) vers la seconde position du à l'effet de la force générée par le matériau mémoire formé.

11. L'appareil de la revendication 9, comprenant en outre un élément de chauffage (35a) disposé de manière adjacente au dispositif de protection distal (39b), et un conducteur électrique ayant une extrémité proximale adaptée à un couplage électrique avec une alimentation externe de courant et une extrémité distale couplée électriquement à l'élément de chauffage (35a), permettant ainsi l'alimentation en énergie de l'élément de chauffage (35a) par un courant électrique lorsque l'extrémité proximale du conducteur électrique est électriquement couplée à l'alimentation externe de courant, la chaleur produite par l'élément de chauffage (35a) entraînant le déplacement du dispositif de protection distal (39b) vers la seconde position par l'effet de la force générée par le matériau mémoire formé.

12. L'appareil de la revendication 8, dans lequel le dispositif de protection distal (19, 39, 39a, 39b) est disposé de manière distante du second orifice (7a, 36b, 66) de l'embout creux (11, 36a, 64).

13. L'appareil de la revendication 8, comportant en outre un membre gonflable (18a, 31, 47) disposé de manière proximale de la partie émettrice de lumière (10, 37, 51), de telle sorte que lorsqu'il est activé, le membre gonflable (18a, 31, 47) et le dispositif de protection distale (19, 39, 48) isole une partie de la lumière corporelle (21, 28) dans lequel l'appareil se trouve disposé.

14. L'appareil de la revendication 13, dans lequel la partie émettrice de lumière (10, 37) peut être positionnée indépendamment du membre gonflable (18a, 31).

15. L'appareil de la revendication 13, dans lequel le dispositif de protection distal comporte un membre gonflable supplémentaire (18a, 31).

16. L'appareil de la revendication 15, dans lequel le dispositif de protection distal (48) et le membre gonflable sont généralement toroïdal.

17. L'appareil de la revendication 15, comportant en outre un manchon non poreux généralement transmettant la lumière (49) s'étendant entre le membre gonflable (47) et le dispositif de protection distal (28), le manchon (49) définissant un conduit au travers duquel un flux de fluide corporel peut être dévié lorsque l'appareil est disposé dans une lumière corporelle (28) et que le membre gonflable (47) et le dispositif de protection distal (48) sont activés.

18. L'appareil de la revendication 17, dans lequel la partie émettrice de lumière (51) est recouverte par le manchon (49).

19. L'appareil de la revendication 17, dans lequel la partie émettrice de lumière (51) est recouverte d'un membre (52) extensible, généralement transmetteur de la lumière, le membre extensible (52) étant englobé par le manchon (49), de sorte que lorsque le membre extensible (52) est activé, tout écoulement de fluide au travers le conduit délimité par le manchon (49) est occulté.

20. L'appareil de la revendication 17, dans lequel la lumière d'écoulement (42) est couplée à au moins un port (42a) pour un écoulement de fluide avec une partie (54) d'une lumière corporelle (28) qui est isolée lorsque l'appareil est disposé à l'intérieur de la lumière corporelle (28) et que le membre gonflable (47) et le dispositif de protection distal (48) sont activés.
